# EUROPÄISCHE PATENTSCHRIFT

(11) **EP 1 481 641 B1**
(45) Veröffentlichungstag und Bekanntmachung des Hinweises auf die Patenterteilung: **22.02.2006**
(21) Anmeldenummer: 04006624.3
(22) Anmeldetag: 19.03.2004
(51) Int. Cl.: A61B 17/34

(54) **Klappenventil für ein Trokarsystem**
Trocar flapper valve
Soupape à clapet pour trocart

(30) Priorität: 30.05.2003 DE 10324684
(43) Veröffentlichungstag der Anmeldung: 01.12.2004
(73) Patentinhaber: Gebrüder Pajunk Besitzverwaltung OHG., 78187 Geisingen (DE)
(72) Erfinder: Pajunk, Heinrich, 78187 Geisingen (DE); Pajunk, Horst, 78187 Geisingen (DE)
(74) Vertreter: Patentanwälte Westphal, Mussgnug & Partner

(56) Entgegenhaltungen:
- EP-B- 0 323 018
- DE-A- 3 923 243
- DE-C- 19 712 726
- US-B1- 6 228 061

## Beschreibung

Die Erfindung betrifft ein Klappenventil für ein Trokarsystem gemäß dem Oberbegriff des Anspruchs 1.

Trokarsysteme werden in der minimal invasiven Chirurgie dazu verwendet, einen operativen Zugang in eine Körperhöhle, z.B. das Abdomen zu schaffen. Hierzu wird mit einem in ein Führungsrohr eingesetzten Trokar die Bauchdecke durchstoßen. Anschließend wird der Trokar abgezogen und das Führungsrohr verbleibt als Instrumentenkanal in der Bauchdecke. Um in der Bauchhöhle operative Eingriffe durchführen zu können, wird in der Regel die Bauchhöhle insuffliert. Um das Entweichen des Gases aus der Bauchhöhle zu verhindern, ist am extrakorporal verbleibenden proximalen Ende des Führungsrohres ein Ventil angeordnet, welches automatisch schließt, wenn kein Instrument in das Führungsrohr eingeführt ist.

Um das Einführen empfindlicher Instrumente zu ermöglichen und ein Verhaken der Instrumente beim Herausziehen zu verhindern, werden Klappenventile verwendet, die manuell geöffnet werden können. Bei diesen Klappenventilen sitzt eine Klappe mit einem Ventilteil unter Federvorspannung schließend auf einer Durchtrittsöffnung, die axial mit dem Führungsrohr fluchtet. Die Klappe ist gegen die Federkraft um eine zur Achse der Durchtrittsöffnung senkrechte außermittige Schwenkachse schwenkbar.

Aus der US 4, 654,030 ist es bekannt, die Klappe drehfest auf der Schwenkachse anzubringen und auf dem aus dem Gehäuse des Ventils herausgeführten Ende der Schwenkachse einen Schwenkhebel für die manuelle Betätigung der Klappe anzubringen. Die. Betätigung des Klappenventils ist ungünstig, da der Operateur mit einer Hand das Trokarsystem halten muss und die zweite. Hand zum Verschwenken der Klappe benötigt.

In der DE 3923243 C2 ist ein Klappenventil gemäß dem Oberbegriff des Anspruchs 1 offenbart. Bei diesem Klappenventil wird die Klappe mittels eines Stößels in die Offenstellung geschwenkt. Der Stößel ist achsparallel zur Achse der Durchtrittsöffnung verschiebbar. Zum Betätigen der Klappe greift der Stößel mit seinem gehäuseinneren Ende an der Klappe zwischen deren Ventilteil und deren Schwenkachse an. Der für den Stößel benötigte Raum vergrößert die radialen und axialen Abmessungen des Gehäuses des Klappenventils. Da aus Platzgründen der Stößel möglichst dicht an der Mittelachse des Gehäuses angeordnet sein muss, ist die Betätigung des Stößels ergonomisch ungünstig.

Der Erfindung liegt die Aufgabe zugrunde ein Klappenventil für ein Trokarsystem zu schaffen, welches einen kompakten Aufbau und eine ergonomisch günstige Handhabung ermöglicht.

Diese Aufgabe wird erfindungsgemäß gelöst durch ein Klappenventil mit den Merkmalen des Anspruchs 1.

Vorteilhafte Ausführungen der Erfindung sind in den Unteransprüchen angegeben.

Bei dem erfindungsgemäßen Klappenventil ist der die Klappe betätigende Stößel radial verschiebbar in das Gehäuse geführt. Das Klappenventil kann daher in einfacher Weise durch Druck auf einen radial angeordneten Druckknopf geöffnet werden. Dadurch ist die Handhabung des Trokarsystems und die Betätigung des Klappenventils mit einer Hand in ergonomisch günstiger Weise möglich. Das Gehäuse des Klappenventils und damit das Trokarsystem kann beispielsweise zwischen dem Zeigefinger und dem Mittelfinger der Hand gehalten werden, während die radiale Betätigung des Klappenventils mittels des Daumens erfolgt. Die Klappe ist als zweiarmiger Hebel ausgebildet. Der Stößel greift an einer Betätigungsfläche an, die an einem Arm der Klappe ausgebildet ist. Der in Bezug auf die Schwenkachse andere Arm der Klappe weist das Ventilteil auf. Die Betätigungsfläche ist vorzugsweise unmittelbar im Bereich der Schwenkachse ausgebildet. Dadurch beansprucht die Betätigung der Klappe keine zusätzlichen radialen Raum, so dass der Außendurchmesser des Gehäuses minimal gehalten werden kann. Außerdem ist ein Verschwenken der Klappe um 90° zwischen der Schließstellung und der Offenstellung mit einem geringen radialen Hubweg des Stößels möglich, was weiter für den kompakten Aufbau des Klappenventils von Vorteil ist. Insbesondere erlaubt der radiale Stößel eine erhebliche Reduzierung der axialen Abmessung des Klappenventils.

In einer vorteilhaften Ausführung ist die Betätigungsfläche der Klappe, an welcher der Stößel angreift in der Weise als Kurve ausgebildet, dass der Bereich, an welchem der Stößel anliegt, in jeder Schwenkstellung der Klappe unter einem ausreichend großen Winkel zur Stößelachse verläuft, um ein Drehmoment in Schwenkrichtung auf die Klappe auszuüben. Die Betätigungsfläche schiebt sich bei der Schwenkbewegung der Klappe gewissermaßen unter dem gehäuseinneren Ende des Stößels vorbei. Über den gesamten linearen Hubweg des Stößels und damit den gesamten Schwenkbereich der Klappe ergibt sich ein im Wesentlichen konstanter Verlauf der Kraft und des auf die Klappe ausgeübten Drehmoments. Dies ist für eine ergonomische Betätigung des Klappenventils von wesentlichem Vorteil.

Im Folgendem wird die Erfindung anhand eines in der Zeichnung dargestellten Ausführungsbeispiels näher erläutert.

Es zeigen
- Figur 1: eine Seitenansicht des Trokarsystems,
- Figur 2: eine axiale Ansicht des Trokarsystems vom proximalen Ende,
- Figur 3: einen Axialschnitt des Trokarsystems,
- Figur 4: eine axiale Ansicht des Klappenventils von der distalen Seite,
- Figur 5: einen Axialschnitt durch das Klappenventil in der Schließstellung,
- Figur 6: einen Axialschnitt durch das Klappenventil in der Offenstellung,
- Figur 7: in vergrößerter Darstellung die Klappe in Seitenansicht und
- Figur 8: eine entsprechende Darstellung der Klappe in Draufsicht.

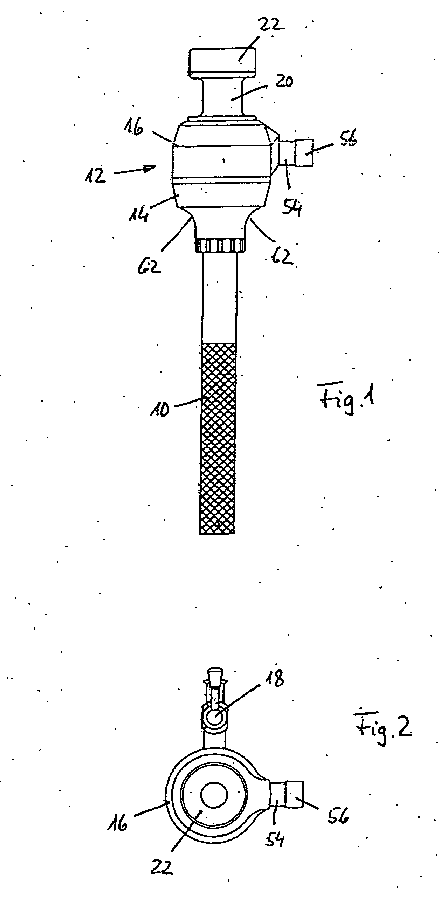

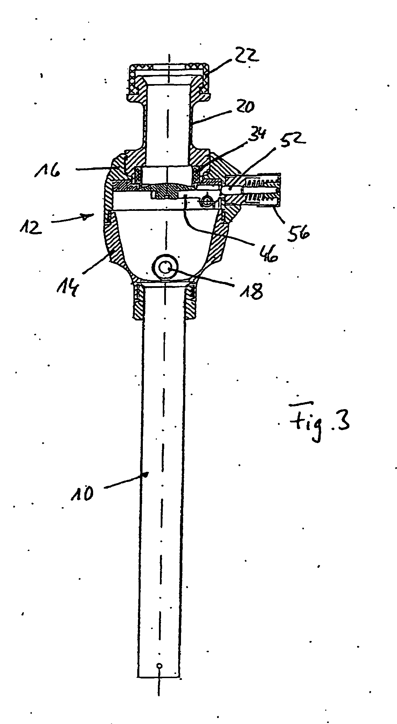

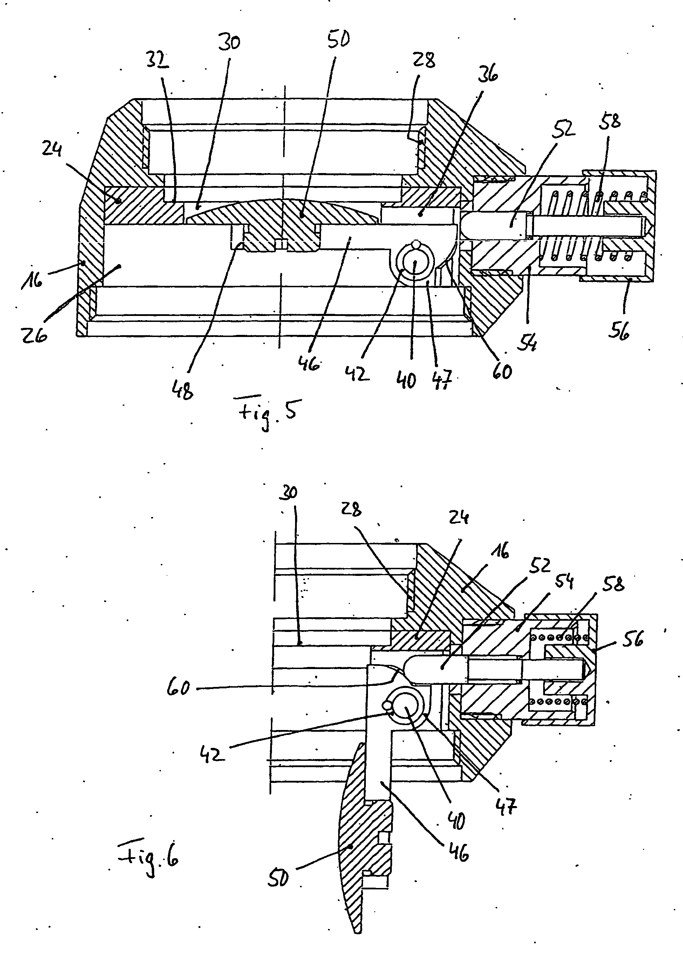

Das in den Figuren 1 bis 3 dargestellte gesamte Trokarsystem weist ein Führungsrohr 10 auf, welches mittels eines eingesetzten und in der Zeichnung nicht dargestellten Trokars als Instrumentenkanal und Zugang für die minimalinvasive Operation z.B. durch die Bauchdecke geführt wird.

Auf das proximale Ende des Führungsrohres 10 wird axial fluchtend ein Gehäuse 12 aufgesetzt, welches aus einem Gehäuseunterteil 14 und einem Klappengehäuse 16 zusammengesetzt ist. In das Gehäuseunterteil führt ein Insufflationshahn 18, über welchen Gas in die Bauchhöhle geleitet werden kann. Das Klappengehäuse 16 nimmt das später im Einzelnen beschriebene Klappenventil auf. Proximal in das Klappengehäuse 1.6 ist abgedichtet eine Einführungshülse 20 eingeschraubt, die axial mit dem Führungsrohr 10 fluchtet und den selben Innendurchmesser aufweist. Auf das proximale Ende der Einführungshülse 20 ist eine weichelastische Gummiabdeckung 22 aufgesetzt, die eine zentrische Öffnung aufweist, deren Durchmesser etwas kleiner ist als der Innendurchmesser der Einführungshülse 20.

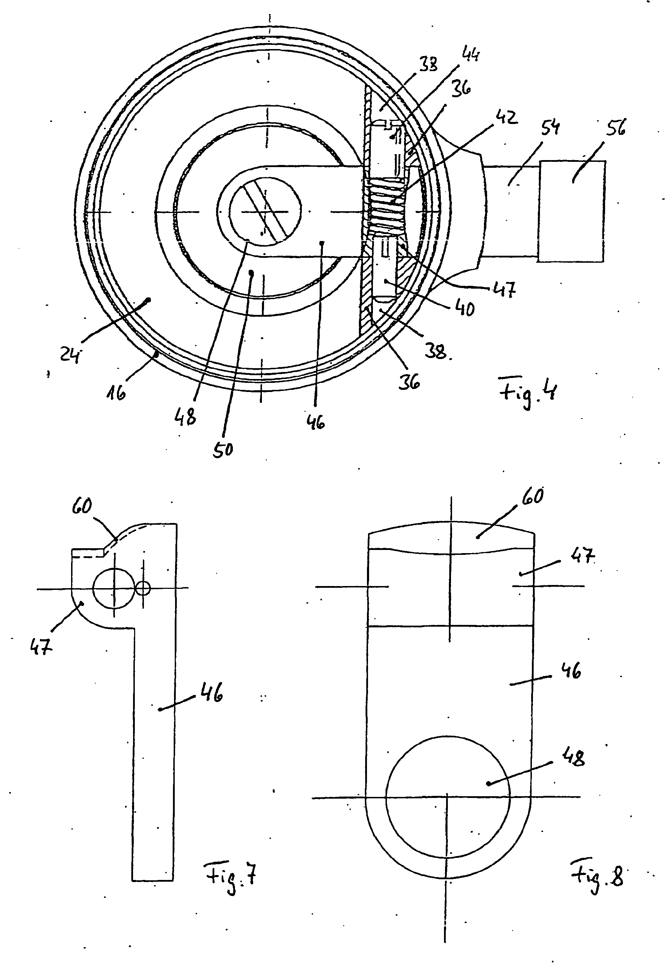

In den Figuren 4 bis 6 ist das Klappengehäuse 16 mit dem in diesem angeordneten Klappenventil im Einzelnen dargestellt. Das Klappengehäuse 16 weist eine distale sich an das Gehäuseunterteil 14 anschießende zylindrische Kammer 26 auf, in welcher eine Klappenaufnahme 24 eingepasst ist. Die Kammer 26 geht koaxial in eine Öffnung über, welche ein Innengewinde 28 aufweist, in welches die Einführungshülse 20 eingeschraubt wird. Die Klappenaufnahme 24 weist eine Durchtrittsöffnung 30 auf, deren Innendurchmesser geringer ist als der Durchmesser der Öffnung des Klappengehäuses 16. Dadurch bildet die Klappenaufnahme 24 eine Innenschulter 32. Eine elastische Dichtungsbuchse 34 ist axial zwischen die Klappenaufnahme 24 und die Einführungshülse 20 eingesetzt und wird zwischen der Innenschulter 32 der Klappenaufnahme 24 und einer Innenschulter der Einführungshülse 20 festgelegt, wie in Figur 3 zu sehen ist. Die Dichtungsbuchse 34 greift koaxial in die Durchtrittsöffnung 30 der Klappenaufnahme 24 und bildet den Ventilsitz für das Klappenventil.

Außerhalb des Außendurchmessers der Durchtrittsöffnung 30 sind an der Klappenaufnahme 24 zwei Lagerklötze 36 angeformt. Zwischen den beiden Lagerklötzen 36 bleibt ein radial angeordneter Aufnahmeraum frei. Die beiden Lagerklötze 36 weisen jeweils eine in Sekantenrichtung verlaufende fluchtende Lagerbohrung 38 auf. In die Lagerbohrung 38 des einen Lagerklotzes 36 ist ein Lagerstift 40 eingesetzt. Auf den Lagerstift 40 ist eine Schenkelfeder 42 koaxial aufgesetzt. Die Schenkelfeder 42 wird durch einen Lagerbolzen 44 gehalten, der in der Lagerbohrung 38 des anderen Lagerklotzes 36 sitzt und koaxial auf den Lagerstift 40 aufgeschraubt wird. Der Lagerstift 40 und der Lagerbolzen 44 lagern schwenkbar eine Klappe 46. Die Klappe 46 ist mit einem angeformten Block 47 zwischen den Lagerklötzen 36 aufgenommen, der in einer mit den Lagerbohrungen 38 fluchtenden Durchgangsbohrung den Lagerstift 40 mit der Schenkelfeder 22 aufnimmt. Die Schenkelfeder 22 ist mit einem Schenkel in der Klappenaufnahme 24 und mit ihrem anderen Schenkel in der Klappe 46 festgelegt. Die Schenkelfeder 42 erteilt dadurch der Klappe 46 eine Vorspannung in der Schwenkrichtung, in welcher die Klappe 46 gegen die Klappenaufnahme 24 geschwenkt wird.

Die Klappe 46, die in den Figuren 7 und 8 als Einzelteil dargestellt ist, besteht aus einer im Wesentlichen rechteckigen Platte, die vom Innenumfang der Kammer 26 radial nach innen verläuft und bis über die Mittelachse des Klappengehäuses 16 ragt. Am radial äußeren Rand der Klappe 46 ist an deren distaler Fläche der Block 47 angeformt. Konzentrisch zur Mittelachse des Klappengehäuses 16 und der Durchtrittsöffnung 30 weist die Klappe 46 eine Aufnahmebohrung 48 auf, in welcher ein kugelkalottenförmiges Ventilteil 50 eingeschraubt ist. Das Ventilteil 50 kommt zur dichtenden Anlage an der Dichtungsbuchse 34, wenn die Klappe 46 durch die Schenkelfeder 42 in die in Figur 5 gezeigte Schließstellung geschwenkt wird. Die Klappe 46 kann gegen die Kraft der Schenkelfeder 42 aus der in Figur 5 gezeigten Schließstellung um 90 ° in die Figur 6 gezeigte Offenstellung geschenkt werden. In dieser Offenstellung ist die Klappe 46 mit dem Ventilteil 50 in distaler Richtung vollständig aus dem Querschnitt der Durchtrittsöffnung 30 herausgeschwenkt. Es kann in dieser Offenstellung ein Instrument durch die Einführungshülse 20, die Durchtrittsöffnung 30 mit der Dichtungsbuchse 34 und das Führungsrohr 10 in das Operationsfeld eingeführt werden. Das Instrument wird proximal durch die Gummiabdeckung 22 abgedichtet. Das Instrument kann ein Operationsinstrument, ein Endoskop oder dergleichen sein.

Die Verschwenkung der Klappe 46 aus der Schließstellung in die Offenstellung erfolgt durch einen Stößel 52. Der Stößel 52 ist radial durch das Klappengehäuse 16 geführt und ist in radialer Richtung und in einer zur Mittelachse des Klappengehäuses 16 senkrechten Ebene verschiebbar. Der Stößel 52 ist in einer Buchse 54 geführt, die radial von außen in das Klappengehäuse 16 eingeschraubt wird. Die Buchse 54 wird an ihrem äußeren Ende von einem Druckknopf 56 koaxial umschlossen. Der Druckknopf 56 ist auf das radial äußere Ende des Stößels 52 aufgepresst. Eine zu dem Stößel 52 koaxiale Schraubendruckfeder 58 ist axial zwischen die Buchse 54 und den Druckknopf 56 eingesetzt und spannt den Druckknopf 56 und den mit diesem fest verbundenen Stößel 52 in die in Figur 5 gezeigte Ruhestellung vor, in welcher der Stößel 52 aus der Kammer 26 des Klappengehäuses 16 radial herausgezogen ist. Durch manuellen Druck auf den Druckknopf 56 kann der Stößel 52 gegen die Kraft der Schraubendruckfeder 58 radial in das Klappengehäuse 16 eingedrückt werden, um die Klappe 56 zu betätigen und aus der in Figur 5 gezeigten Schließstellung in die in Figur 6 gezeigte Offenstellung zu verschwenken.

Um die Klappe 46 mittels des Stößels 52 zu verschwenken, ist die Klappe 46 in folgender Weise ausgebildet. Die durch den Lagerstift 40 und den Lagerbolzen 44 gebildete Schwenkachse der Klappe 46 verläuft durch den Block 47 und ist gegen die Ebene der Klappe 46 distal versetzt. Die Ebene der Klappe 46 verläuft daher außermittig im Bezug auf die Schwenkachse. Die Achse des Stößels 52, die mit der Richtung seine Linearbewegung zusammenfällt, ist so ausgerichtet, dass sie nahezu mit der proximalen Oberseite der Klappe 46 zusammenfällt, wenn sich die Klappe 46 in der in Figur 5 gezeigten Stellung befindet. An der Klappe 46 ist ein Betätigungsfläche 60 ausgebildet, an welcher der Stößel 52 mit seinem gehäuseinneren Ende angreift. Diese Betätigungsfläche 60 ist am deutlichsten in den Figuren 7 und 8 zu erkennen. Die Betätigungsfläche 60 ist an dem dem Ventilteil 50 entgegengesetzten radial äußeren Ende der Klappe 46 angeordnet. Die Betätigungsfläche 60 beginnt an der proximalen Aussenkante der Klappe 46, verläuft in distaler Richtung bis in den Block 47 und nähert sich dabei zunehmend der durch den Lagerstift 40 bestimmten Schwenkachse. Der radiale Abstand der Betätigungsfläche 60 von der Schwenkachse 40 nimmt somit von der proximalen Seite der Klappe 46 gegen den Block 47 hin ab. Zu Beginn der Öffnungsschwenkbewegung der Klappe 46 drückt der Stößel 52 an der proximalen Seite der Klappe 46 parallel zu dieser gegen einen Bereich der Betätigungsfläche 60, der senkrecht zur Klappenebene verläuft. Am Ende der Öffnungsschwenkbewegung drückt der Stößel 52 dagegen senkrecht zur Klappenebene gegen einen Bereich der Betätigungsfläche 60, der nahezu parallel zur Klappenebene verläuft. Dadurch wird die lineare Bewegung des Stößels 52 in eine Winkelschwenkbewegung der Klappe 46 umgesetzt, wobei der Stößel während der gesamten Schwenkbewegung nahezu senkrecht an der Betätigungsfläche angreift, und ein optimales Drehmoment auf die Klappe 46 ausübt.

Wie aus den Figuren 7 und 8 ersichtlich, ist die Betätigungsfläche 60 in der zur Schwenkachse 40 parallelen Richtung leicht konkav gewölbt, so dass sich eine gute Gleitfläche für das abgerundete gehäuseinnere Ende des Stößels 52 ergibt.

Eine ergonomisch günstige Handhabung des Trokarsystems ist dadurch möglich, dass am distalen Ende des Gehäuses 12 beiderseits des Führungsrohres 10 jeweils eine Fingermulde 62 eingeformt ist. Das Gehäuse 12 und damit das gesamte Trokarsystem wird gehalten, indem Zeigefinger und Mittelfinger in diese Fingermulden 62 gelegt werden. Der Druckknopf 56 kann dann zur Betätigung des Klappenventils mit dem Daumen derselben Hand gedrückt werden.

### Bezugszeichenliste

- 10: Führungsrohr
- 12: Gehäuse
- 14: Gehäuseunterteil
- 16: Klappengehäuse
- 18: Insufflationshahn
- 20: Einführungshülse
- 22: Gummiabdeckung
- 24: Klappenaufnahme
- 26: Kammer
- 28: Innengewinde
- 30: Durchtrittsöffnung
- 32: Innenschulter
- 34: Dichtungsbuchse
- 36: Lagerklötze
- 38: Lagerbohrungen
- 40: Lagerstift
- 42: Schenkelfeder
- 44: Lagerbolzen
- 46: Klappe
- 47: Block
- 48: Aufnahmebohrung
- 50: Ventilteil
- 52: Stößel
- 54: Buchse
- 56: Druckknopf
- 58: Schraubendruckfeder
- 60: Betätigungsfläche
- 62: Fingermulden

## Patentansprüche

1. Klappenventil für ein Trokarsystem, mit einem Gehäuse (12), welches proximal an ein Führungsrohr (10) ansetzbar ist, mit einer in dem Gehäuse (12) angeordneten mit dem Führungsrohr (10) axial fluchtenden Durchtrittsöffnung (30), mit einer ein Ventilteil (50) aufweisenden Klappe (46), die um eine zur Achse der Durchtrittsöffnung (30) senkrechte außermittige Schwenkachse schwenkbar ist, und mit einem Stößel (52), welcher in dem Gehäuse (12) manuell verschiebbar geführt ist und durch welchen die Klappe (46) aus einer die Durchtrittsöffnung (30) mit ihrem Ventilteil (50) verschließenden Schließstellung gegen eine Federkraft in eine die Durchtrittsöffnung freigebende Offenstellung verschwenkbar ist,
**dadurch gekennzeichnet, dass** der Stößel (52) in einer Linie verschiebbar ist, die in einer zur Achse der Durchtrittsöffnung im Wesentlichen senkrechten Ebene liegt, im Wesentlichen radial zur Achse der Durchtrittsöffnung verläuft und die Schwenkachse der Klappe nicht schneidet, dass die Klappe (46) in Bezug auf ihre Schwenkachse als zweiarmiger Hebel ausgebildet ist, dessen einer Arm das Ventilteil (50) aufweist und an dessen anderem Arm eine Betätigungsfläche (60) ausgebildet ist, an welcher der Stößel (52) mit seinem gehäuseinneren Ende angreift, und dass die Betätigungsfläche (60) so geformt ist, dass in jeder Schwenkwinkelstellung der Klappe (46) die Mittelachse des Stößels (52) mit dem Bereich der Betätigungsfläche (60), an welchem der Stößel (52) anliegt, einen Winkel von wenigstens 15° einschließt.

2. Klappenventil nach Anspruch 1,
**dadurch gekennzeichnet, dass** der Winkel zwischen der Mittelachse des Stößels (52) und der Betätigungsfläche (60) in jeder Schwenkwinkelstellung der Klappe (46) wenigstens 30°, vorzugsweise wenigstens 45° beträgt.

3. Klappenventil nach Anspruch 1 oder 2,
**dadurch gekennzeichnet, dass** die Schwenkachse der Klappe (46) an deren distaler Seite angeordnet ist und dass die Betätigungsfläche (60) proximal von der Schwenkachse an der Klappe (46) ausgebildet ist.

4. Klappenventil nach Anspruch 3,
**dadurch gekennzeichnet, dass** die Klappe (46) die Form einer Platte hat, dass die Schwenkachse einen Block (47) durchsetzt, der an der distalen Seite der Klappe (46) an deren radial äußerem Rand angeformt ist, und dass die Betätigungsfläche (60) von der proximalen Außenkante der Klappe (46) in diesen Block (47) hinein verläuft,

5. Klappenventil nach Anspruch 4,
**dadurch gekennzeichnet, dass** die Mittelachse des Stößels (52) parallel und in geringem Abstand zu der proximalen Fläche der Klappe (46) verläuft, wenn sich die Klappe (46) in ihrer Schließstellung befindet.

6. Klappenventil nach Anspruch 5,
**dadurch gekennzeichnet, dass** die Betätigungsfläche (60) in dem an die proximale Außenkante der Klappe (46) anschließenden Bereich, in welchem der Stößel (52) in der Schließstellung der Klappe (46) anliegt, im Wesentlichen rechtwinklig zur proximalen Fläche der Klappe (46) verläuft, dass sich der radiale Abstand der Betätigungsfläche (60) von der Schwenkachse der Klappe (46) von der proximalen Außenkante der Klappe (46) gegen den Block (47) hin verringert und dass die Betätigungsfläche (60) in ihrem distalen Endbereich, an welchem der Stößel (52) in der Offenstellung der Klappe (46) anliegt, unter einem Winkel von weniger als 45° zur Ebene der Klappe (46) verläuft.

7. Klappenventil nach einem der vorhergehenden Ansprüche,
**dadurch gekennzeichnet, dass** der Stößel (52) in einer radial in das Gehäuse (12, 16) eingesetzten Buchse (54) geführt ist und mittels eines Druckknopfes (56) gegen die Kraft einer Feder (58) zum Öffnen der Klappe (46) radial eindrückbar ist.

8. Klappenventil nach einem der vorhergehenden Ansprüche,
**dadurch gekennzeichnet, dass** distal an dem Gehäuse (12) beiderseits des Führungsrohres (10) seitliche Fingermulden (62) ausgebildet sind.

## Claims

1. A flap valve for a trocar system, comprising a housing (12) which can be placed proximally onto a guide tube (10), a passage opening (30) that is disposed in the housing (12) and axially aligns with the guide tube (10), a flap (46) comprising a valve part (50) and pivotable around an eccentric pivot axis perpendicular to the axis of the passage opening (30), and a tappet (52), which is manually displaceably guided in the housing (12) and by which the flap (46) can be swung out of a closed position closing the passage opening (30) with its valve part (50) against a spring force into an open position uncovering the passage opening,
**characterised in that** the tappet (52) is displaceable in a line which lies in a plane substantially perpendicular to the axis of the passage opening, extends substantially radially to the axis of the passage opening and does not intersect the pivot axis of the flap,
**in that** the flap (46) in relation to its pivot axis is constructed as a two-armed lever, the one arm of which comprises the valve part (50) and on the other arm of which there is provided an actuating face (60), on which the tappet (52) acts with its end located inside the housing,
**and in that** the actuating face (60) is formed so that in each pivot angle position of the flap (46) the centre axis of the tappet (52) encloses an angle of at least 15° with the region of the actuating face (60) against which the tappet (52) rests.

2. A flap valve according to Claim 1,
**characterised in that** the angle between the centre axis of the tappet (52) and the actuating face (60) in each pivot angle position of the flap (46) is at least 30°, preferably at least 45°.

3. A flap valve according to Claim 1 or 2,
**characterised in that** the pivot axis of the flap (46) is disposed at its distal side
**and in that** the actuating face (60) is constructed proximally of the pivot axis on the flap (46).

4. A flap valve according to Claim 3,
**characterised in that** the flap (46) takes the form of a plate,
**in that** the pivot axis passes through a block (47), which at the distal side of the flap (46) is moulded on its radially outer edge,
**and in that** the actuating face (60) extends from the proximal outer edge of the flap (46) into this block (47).

5. A flap valve according to Claim 4,
**characterised in that** the centre axis of the tappet (52) runs parallel and at a slight distance from the proximal face of the flap (46) when the flap (46) is situated in its closed position.

6. A flap valve according to Claim 5,
**characterised in that** in the region following the proximal outer edge of the flap (46) in which the tappet (52) lies in the closed position of the flap (46), the actuating face (60) extends substantially at right angles to the proximal face of the flap (46),
**in that** the radial distance of the actuating face (60) from the pivot axis of the flap (46) is reduced from the proximal outer edge of the flap (46) towards the block (47) **and in that** the actuating face (60) in its distal end region, against which the tappet (52) rests in the open position of the flap (46), extends at an angle of less than 45° to the plane of the flap (46).

7. A flap valve according to one of the preceding Claims,
**characterised in that** the tappet (52) is guided in a bushing (54) inserted radially into the housing (12, 16) and can be radially pushed in by means of a push button (56) against the force of a spring (58) to open the flap (46).

8. A flap valve according to one of the preceding Claims,
**characterised in that** lateral finger depressions (62) are provided distally on the housing (12) on either side of the guide tube (10).

## Revendications

1. Soupape à clapet pour trocart, comprenant
- un boîtier (12) pouvant être mis en place de façon proximale au niveau d'un tube de guidage (10),
- une ouverture de passage (30) disposée dans le boîtier (12) et alignée axialement avec le tube de guidage (10),
- un clapet (46) présentant une partie de soupape (50) et pouvant pivoter autour d'un axe excentré perpendiculaire à l'axe de l'ouverture de passage (30), et
- un poussoir (52) guidé manuellement de façon mobile dans le boîtier (12) et permettant de faire pivoter le clapet (46), d'une position de fermeture de l'ouverture de passage (30) avec sa partie de soupape (50), à l'encontre d'une force de ressort, à une position d'ouverture dégageant l'ouverture de passage,
**caractérisée en ce que**
le poussoir (52) est mobile sur une ligne située dans un plan essentiellement perpendiculaire à l'axe de l'ouverture de passage, qui s'étend pour l'essentiel radialement par rapport à l'axe de l'ouverture de passage et qui ne coupe pas l'axe de pivotement du clapet (46),
le clapet (46) se présente comme un levier à deux bras par rapport à son axe de pivotement, l'un des bras présentant la partie de soupape (50) tandis que l'autre comporte une surface d'actionnement (60) au niveau de laquelle le poussoir (52) s'accroche par son extrémité située à l'intérieur du boîtier, et
la surface d'actionnement (60) est formée de telle sorte que dans chaque position angulaire de pivotement du clapet (46), l'axe médian du poussoir (52) forme, avec la zone de la surface d'actionnement (60) contre laquelle repose le poussoir (52), un angle d'au moins 15°.

2. Soupape à clapet selon la revendication 1,
**caractérisée en ce que**
l'angle formé par l'axe médian du poussoir (52) et la surface d'actionnement (60), dans chaque position angulaire de pivotement du clapet (46), est d'au moins 30°, de préférence d'au moins 45°.

3. Soupape à clapet selon la revendication 1 ou 2,
**caractérisée en ce que**
l'axe de pivotement du clapet (46) se situe à son côté distal et la surface d'actionnement (60) est proximale par rapport à l'axe de pivotement au niveau du clapet (46).

4. Soupape à clapet selon la revendication 3,
**caractérisée en ce que**
le clapet (46) a la forme d'une plaque, l'axe de pivotement traverse un bloc (47) formé au côté distal du clapet (46) sur son arête extérieure radiale, et la surface d'actionnement (60) s'étend à l'intérieur de ce bloc (47) à partir de l'arête extérieure proximale du clapet (46).

5. Soupape à clapet selon la revendication 4,
**caractérisée en ce que**
l'axe médian du poussoir (52) s'étend parallèlement à la surface proximale du clapet (46) et à faible distance de celle-ci lorsque le clapet (46) se trouve en position de fermeture.

6. Soupape à clapet selon la revendication 5,
**caractérisée en ce que**
la surface d'actionnement (60) s'étend pour l'essentiel perpendiculairement à la surface proximale du clapet (46), dans la zone rattachée à l'arête extérieure proximale du clapet (46) où repose le poussoir (52) dans la position de fermeture du clapet (46),
la distance radiale entre la surface d'actionnement (60) et l'axe de pivotement du clapet (46) se rétrécit à partir de l'arête extérieure proximale du clapet (46) contre le bloc (47), et
la surface d'actionnement (60), dans sa zone d'extrémité distale où repose le poussoir (52) dans la position d'ouverture du clapet (46), s'étend en formant un angle inférieur à 45° par rapport au plan du clapet (46).

7. Soupape à clapet selon l'une quelconque des revendications précédentes,
**caractérisée en ce que**
le poussoir (52) est guidé dans une douille (54) introduite radialement dans le boîtier (12, 16) et peut être enfoncé radialement au moyen d'un bouton-poussoir (56) à l'encontre de la force d'un ressort (58) pour l'ouverture du clapet (46).

8. Soupape à clapet selon l'une quelconque des revendications précédentes,
**caractérisée en ce que**
des creux latéraux pour les doigts (62) sont formés des deux côtés du tube de guidage (10) de manière distale au niveau du boîtier (12).
